# EUROPÄISCHE PATENTSCHRIFT

(11) **EP 0 795 543 B1**
(45) Veröffentlichungstag und Bekanntmachung des Hinweises auf die Patenterteilung: **31.10.2001**
(21) Anmeldenummer: 97103444.2
(22) Anmeldetag: 03.03.1997
(51) Int. Cl.: C07C 263/04

(54) **Verwendung von hochsiedenden Lösemitteln bzw. -gemischen als Wärmetauschmedium für die thermische Spaltung von Carbamidsäureestern**
Use of high boiling solvents or solvent mixtures as heat exchange medium in the thermal decomposition of carbamic esters
Utilisation de solvants ou de mélanges de solvants à point d'ébullition élevé comme fluide d'échange de chaleur dans la décomposition thermique d'esters carbamiques

(30) Priorität: 15.03.1996 DE 19610261; 10.05.1996 DE 19618828
(43) Veröffentlichungstag der Anmeldung: 17.09.1997
(73) Patentinhaber: BAYER AG, 51368 Leverkusen (DE)
(72) Erfinder: Schleenstein, Dieter, 51519 Odenthal (DE); Rasp, Christian, Dr., 51467 Bergisch Gladbach (DE); Ronge, Georg, Dr., 40227 Düsseldorf (DE); Wilmes, Oswald, Dr., 51061 Köln (DE)

(56) Entgegenhaltungen:
- EP-A- 0 092 738
- EP-A- 0 323 514
- EP-A- 0 542 106
- DE-A- 4 231 417
- DE-A- 4 413 580
- US-A- 3 919 278
- Beilstein database, Daten zur Verbindung Benzyl-n-butyl-phthalat
- Kirk- Othmer, Encyclopedia Chem. Technol. 4th ed., vol. 4, Seite 225

## Beschreibung

Die Erfindung betrifft die Verwendung von definiert bzw. in einem engen Bereich siedenden inerten thermostabilen Lösemitteln bzw. -gemischen als Wärmetauschmedium für die thermische Spaltung von Carbamidsäureestern (Urethanspaltung).

Es ist bekannt, zur Spaltung von Carbamidsäureestem zu Isocyanaten zwischen Gas- und Flüssigphasenspaltung sowie Spaltung in der Wirbelschicht zu unterscheiden. Die Spaltung in der Gasphase wird zum Beispiel in den EP-A 28 724, EP-A 100 047, EP-A 126 299, EP-A 126 300, EP-A 143 120, EP-A 261 604, EP-A 449 110 und in den US-A 3 734 941 und 3 870 739 beschrieben. Die Spaltung in der Gasphase ist ein Hochtemperatur-Verfahren, was in der Regel bei Temperaturen >300°C im Vakuum <25 mbar durchgeführt wird. Der hiermit verbundene verfahrenstechnische Aufwand, die thermische Belastung der Edukte und Produkte, die notwendige Vorab-Verdampfung des Carbamidsäureesters sowie die zum Teil noch ungeklärten katalytischen Effekte von Metalloberflächen bewirken, daß die Gasphasenspaltung gegenüber der Spaltung in der Flüssigphase nachteilig ist. Insbesondere steht eine Verstopfungsgefahr im Verdampferbereich durch Verkrustung, da das Problem der Ausschleusung höhermolekularer Folgeprodukte nicht gelöst ist.

Die Spaltung in der Wirbelschicht wird zum Beispiel in der EP-A 78 005 beschrieben. Diese Verfahren sind mit hohem Energieeinsatz behaftet und lassen Schwierigkeiten bei der technischen Realisierung erwarten, so daß ein Einsatz aufgrund des vorläufigen Entwicklungsstandes im technischen Maßstab nicht absehbar ist.

Die Spaltung in der Flüssigphase ermöglicht im Vergleich zur Gasphasenspaltung niedrige Reaktionstemperaturen von <300°C, erfordert aber das schnelle Abtrennen der Reaktionsprodukte.

Zum einen wird dadurch die Rückreaktion von Isocyanat und Hydroxylkomponente zum Carbamidsäureester vermieden, zum anderen wird die Bildung von harzartigen Nebenprodukten, die zu Ablagerungen in den Apparaten führen können, vermindert bzw. vermieden. Durch Verdünnen mit einem inerten Lösemittel kann die Entstehung höhermolekularer Folgeprodukte vermindert werden, gleichzeitig dient das Lösemittel zur Ausschleusung dieser Nebenkomponenten. Die nachfolgend aufgeführten Verfahren können nach Art des Reaktors in Rührreaktor (EP-A 355 443), Dünnschichter bzw. Rohrreaktor (EP-A 61 013, EP-A 92 738, EP-A 396 977), Reaktor mit aufgsetzter Kolonne (EP 323 514, EP-A 524 554), kombinierte Spalt- und Rektifizierkolonne (EP-A 568 782) und in Reaktionskolonnen (EP-A 542 106) unterschieden werden. Ein weiteres Unterscheidungsmerkmal ist die Gegenwart oder der Verzicht auf ein Lösemittel bei der Spaltreaktion.

Die lösemittelfreie Spaltung wird in EP-A 355 443, EP-A 568 782, EP-A 966 925 und EP-A 524 554 beschrieben. Nachteilig ist hier, daß die Spaltung im Sumpf, d.h. im Verdampfer abläuft. In diesem beheizten Bereich besteht aufgrund der starken Temperaturgradienten die Gefahr der Nebenproduktbildung. Um diese Nebenprodukte zu entfernen, müssen bezogen auf den Einsatz hohe Anteile (15 bis 25 Gew.-%) ausgeschleust werden. Andernfalls können Anbackungen auftreten, die bis zur Verstopfung des Reaktors führen.

Durch Lösemittelzusatz wird das Problem der Anbackungen zurückgedrängt (EP-A 61 013, EP-A 92 738, EP-A 323 514, EP-A 542 106). Die besten Ausbeuten lassen sich hier erzielen, wenn die Spaltung im Abtriebsteil einer kombinierten Spalt- und Rektifizierkolonne durchgeführt wird.

Mit Hilfe eines geeigneten, schwersiedenden Lösemittels wird verhindert, daß die Reaktion im Verdampferbereich abläuft, wobei das Lösemittel durch Verdampfung und Kondensation die Wärmeenergie vom Verdampfer in die Reaktionszone transportiert.

Die Spaltung des Carbamidsäureesters kann in einer derartigen Spalt- und Rektifizierkolonne so betrieben werden, daß kein Carbamidsäureester mit den Heizflächen im Verdampfer in Berührung kommt und somit lange Betriebszeiten der Apparatur möglich sind. Im Gegensatz zu der in der DE-A 4 231 417 beschriebenen Kolonne und auch dem in EP-A 0 524 554 beschriebenem Reaktor ist nur eine geringe Ausschleusung vom Sumpfinhalt notwendig, da an dieser Stelle weder Carbamidsäureester noch Spalt- bzw. Nebenprodukte analytisch nachweisbar sind.

Durch das Verfahren gelingt es, eine vollständige, nebenproduktfreie Spaltung des Carbamidsäureesters im Destillationsteil zu erreichen. Dies hat zur Folge, daß durch die vollständige Spaltung Ausbeuteverluste vermieden werden, daß die nachfolgende Aufarbeitung deutlich vereinfacht ist und daß Nebenreaktionen und Anbackungen im Sumpf vermieden werden, weil reagierende Produkte gar nicht in den Sumpf gelangen, so daß die Standzeiten entscheidend verlängert werden.

Bei den beim erfindungsgemäßen Verfahren einzusetzenden Carbamidsäureestern handelt es sich um Verbindungen der allgemeinen Formel R¹(NHCOOR²)ₙ.

In dieser Formel stehen
- R¹: für einen gegebenenfalls inerte Substituenten aufweisenden aliphatischen Kohlenwasserstoffrest mit insgesamt 4 bis 10 Kohlenstoffatomen, einen gegebenenfalls inerte Substituenten aufweisenden cycloaliphatischen Kohlenwasserstoffrest mit insgesamt 6 bis 15 Kohlenstoffatomen, einen gegebenenfalls inerte Substituenten aufweisenden araliphatischen Kohlenwasserstoffrest mit insgesamt 7 bis 10 Kohlenstoffatomen oder einen gegebenenfalls inerte Substituenten aufweisenden aromatischen Kohlenwasserstoffrest mit insgesamt 6 bis 15 Kohlenstoffatomen.
- R²: für einen aliphatischen Kohlenwasserstoffrest mit 1 bis 20 Kohlenstoffatomen, einen cycloaliphatischen Kohlenwasserstoffrest mit 5 bis 15 Kohlenstoffatomen oder einen aromatischen Kohlenwasserstoffrest mit 6 bis 15 Kohlenstoffatomen und
- n: für eine ganze zahl von 2 bis 5.

Vorzugsweise werden beim erfindungsgemäßen Verfahren solche Carbamidsäureester der genannten Formel eingesetzt, für welche
- R¹: für einen aliphatischen Kohlenwasserstoffrest mit insgesamt 4 bis 12, insbesondere 5 bis 10 Kohlenstoffatomen, einen cycloaliphatischen Kohlenwasserstoffrest mit 6 bis 15 Kohlenstoffatomen, einem Xylylenrest oder einen aromatischen, gegebenenfalls Methylsubstituenten und/oder Methylenbrücken aufweisenden Kohlenwasserstoffrest mit insgesamt 6 bis 15 Kohlenstoffatomen steht,
- R²: für einen aliphatischen Kohlenwasserstoffrest mit 1 bis 6, insbesondere 1 bis 4 Kohlenstoffatomen, einen Cyclohexylrest oder einen Phenylrest steht, und
- n: für eine ganze Zahl von 2 bis 4 steht.

Beispiele für geeignete Carbamidsäureester sind
1-(Butoxycarbonylamino)-3,3,5-trimethyl-5-(butoxycarbonylamino-methyl)-cyclohexan,
1-(Methoxycarbonylamino)-3,3,5-trimethyl-5-(methoxycarbonylamino-methyl)-cyclohexan,
1-Methyl-2,4-bis-(methoxycarbonylamino)-benzol,
1-Methyl-2,6-bis-(methoxycarbonylamino)-benzol,
1-Methyl-2,4-bis-(butoxycarbonylamino)-benzol,
1-Methyl-2,6-bis-(butoxycarbonylamino)-benzol,
1,10-Bis-(methoxycarbonylamino)-decan,
1,12-Bis-(butoxycarbonylamino)-dodecan,
1,12-Bis-(methoxycarbonylamino)-dodecan,
1,12-Bis-(phenoxycarbonylamino)-dodecan,
1,3-Bis-(ethoxycarbonylamino-methyl)-benzol,
1,3 -Bis-(methoxycarbonylamino)-benzol,
1,3 -Bis-[(methoxycarbonylamino)-methyl]-benzol,
1,3,6-Tris-(methoxycarbonylamino)-hexan,
1,3,6-Tris-(phenoxycarbonylamino)-hexan,
1,4-Bis-(ethoxycarbonylamino)-butan,
1,4-Bis-(ethoxycarbonylamino)-cyclohexan,
1,5-Bis-(butoxycarbonylamino)-naphthalin,
1,6-Bis-(methoxycarbonylamino)-hexan,
1,6-Bis-(ethoxycarbonylamino)-hexan,
1,6-Bis-(butoxycarbonylamino)-hexan,
1,5-Bis-(methoxycarbonylamino)-pentan,
1,6-Bis-(methoxymethylcarbonylamino)-hexan,
1,8-Bis-(ethoxycarbonylamino)-octan,
1,8-Bis-(phenoxycarbonylamino)-4-(phenoxycarbonylaminmethyl)-octan,
2,2'-Bis-(4-propoxycarbonylamino-phenyl)-propan,
2,4'-Bis-(ethoxycarabonylamino)-diphenylmethan,
2,4-Bis-(methoxycarbonylamino)-cyclohexan,
4,4'-Bis-(ethoxycarbonylamino)-dicyclohexylmethan,
2,4'-Bis-(ethoxycarbonylamino)-diphenylmethan,
4,4'-Bis-(methoxycarbonylamino)-2,2'-dicyclohexylpropan,
4,4'-Bis-(methoxycarbonylamino)-biphenyl,
4,4'-Bis-(butoxycarbonylamino)-2,2'-dicyclohexylpropan,
4,4'-Bis-(phenoxycarbonylamino)-dicyclohexylmethan,
4,4'-Bis-()phenoxycarbonylamino)-diphenylmethan.

Bei den genannten "Butoxy-Resten" handelt es sich um iso- und um n-Butoxy-Reste.

Als geeignete Lösemittel für die Durchführung der Spaltung in derartigen Kolonnen kommen z.B. gemäß der EP-A 542 106 Flüssigkeiten oder Feststoffe in Betracht, deren Siedepunkte unter den Druckbedingungen im Sumpf der Kolonne mindestens 10°C, vorzugsweise mindestens 40°C oberhalb der Siedepunkte der dem zu spaltenden Carbamidsäureestern zugrundeliegenden Isocyanate und Alkohole liegen und die folgende Bedingungen erfüllen:
a) sie lösen unter den Spaltbedingungen weitgehend sowohl die als Ausgahgsmaterialien eingesetzten Carbamidsäureester als auch die Reaktionsnebenprodukte anfallenden Isocyanatfolgeprodukte,
b) sie sind unter den Spaltbedingungen thermisch weitgehend stabil,
c) sie sind gegenüber den eingesetzten Carbamidsäureestern und den entstehenden Isocyanaten weitgehend chemisch inert,
d) sie sind unter den Spaltbedingungen weitgehend destillierbar,
e) sie können destillativ von den Reaktionsnebenprodukten weitgehend abgetrennt werden,
f) sie sind weitgehend recyclisierbar.

Diesen Forderungen enstprechend kommen als erfindungsgemäß verwendbare Hochsieder beispielsweise die in US-A 3 919 278, EP-A 323 514, EP-A 61 013 und EP 92 738 erwähnten Verbindungen, außerdem beispielsweise die verschiedenen, isomeren Benzyltoluole, Terphenyle, Phenoxybiphenyle, Phthalsäure-di(ar)-alkylester, o-Phosphorsäure-tri(ar)alkylester mit jeweils 1 bis 10 Kohlenstoffatomen in den (Ar)alkylestern oder beliebige Gemische derartiger Verbindungen in Betracht.
Für den Einsatz in den erwähnten Spalt- und Rektifizierkolonnen eignen sich besonders technisches Dibenzyltoluol, Benzyl-n-butyl-phthalat, technisches Terphenyl bzw. teilhydrierte Terphenyle, Phenoxybiphenyle bzw. deren Isomerengemische. Nachteilig ist jedoch, daß handelsübliche hochsiedende Lösemittel bzw. Wärmeträger keinen definierten Siedepunkt, sondern einen Siedebereich aufweisen. Dies führt in der Spaltkolonne zu einer destillativen Trennung der Lösemittelmischung und damit zu einem breiten Temperaturprofil, im Extremfall destillieren leichtsiedende Bestandteile mit den Spaltprodukten Isocyanat bzw. Alkohol ab, die höhersiedenden Produkte reichern sich im Sumpf der Kolonne an.

Aufgabe der Erfindung war es daher, geeignete Lösemittel bzw. Lösemittelgemische für die thermische Spaltung in nachfolgende näher beschriebenen kombinierten Spalt- und Rektifizierkolonnen zur Verfügung zu stellen. Mit Hilfe eines geeigneten, schwersiedenden Lösemittels wird verhindert, daß die Reaktion im Verdampferbereich abläuft, wobei das Lösemittel durch Verdampfung und Kondensation die Wärmeenergie vom Verdampfer in die Reaktionszone transportiert und somit als Wärmetauschmedium fungiert. Diese Hochsieder sollten im Gegensatz zu hochtemperaturbeständigen handelsüblichen Wärmeträgern einen möglichst engen Siedebereich (Temperaturfenster) aufweisen, um so die Wahl eines "Temperaturfensters" zu ermöglichen, in dem einerseits die Temperatur niedrig genug ist, daß die Nebenproduktbildung nicht oder nur schwach einsetzt, und in dem andererseits die Temperatur hoch genug ist, daß die Spaltung schon ausreichend schnell verläuft. Die Einstellung des gewünschten "Temperaturfensters" im Reaktionsteil erfolgt über die Einstellung des Absolutdrucks im Reaktionsteil. Ein engsiedendes Lösemittel ist bevorzugt dann einzusetzen, wenn dieses verfügbare "Temperaturfenster" für den gewählten Carbamidsäureester eng ist, andernfalls läßt sich die Spaltung auch bei Einsatz der bislang verwendeten weitsiedenden Wärmeträger durchführen.

Überraschenderweise konnte nun gefunden werden, daß ein solches optimal angepaßtes "Spaltmedium" mit scharfem Siedepunkt in der Tat das Temperaturgefälle in einer Spaltkolonne erniedrigt und somit im Vergleich mit Spalt- bzw. Wärmetauschmedien mit einem Siedebereich bei gleicher Spaltleistung die Sumpftemperaturen abzusenken erlaubt. Dies wiederum unterstützt die Vermeidung einer möglichen Höhersiederbildung im Spaltsumpf und macht die Spaltung technisch beherrschbar.

Gegenstand der Erfindung ist die Verwendung eines Lösemittels, welches einen definierten Siedepunkt (Reinstoff) oder aber einen engen Siedebereich < 10°C, bevorzugt < 6°C, besonders bevorzugt < 3°C bei Betriebsdruck aufweist, und welches als Destillationsschnitt von thermostabilen Flüssigkeiten gewonnen wird, ausgewählt aus der Gruppe ortho-, meta- und para- Isomeren von Phenoxybiphenyl zur thermischen Spaltung von Carbamidsäureestern.

Durch die Verwendung von engsiedenden Destillationsschnitten konnte die Temperaturdifferenz im Reaktionsteil einer nachstehend beschriebenen Spaltkolonne bei gegebener Belastung (d.h. gegebener Druckverlust) von 16°C (Marlotherm S) auf 7,5°C (ortho-Phenoxybiphenyl) reduziert werden. Folglich konnte die Sumpftemperatur der Kolonne (bei gleicher Spaltleistung und unveränderter mittlerer Temperatur im Reaktionsteil) um 4°C verringert werden, wodurch die Gefahr der Bildung von Neben- und Crackprodukten im Sumpf deutlich reduziert wurde. Crackprodukte im Sumpf können entstehen, wenn Carbamidsäureester aufgrund von technischen Störungen in den Sumpf gelangen und weiterreagieren.

Wesentlich ist der Ablauf der Reaktion als Reaktivrektifikation mit dem inerten Lösemittel. Der Lösemitteldampf steigt vom Verdampfer auf und stellt im Abtriebsteil durch Kondensation die Energie für die endotherme Reaktion sowie für die teilweise Verdampfung zur Verfügung.

Die Spaltprodukte steigen dampfförmig auf und werden somit unmittelbar der Reaktion in der Flüssigphase entzogen. Bei der Spaltung von mehrfunktionellen Carbamidsäureestern kann in dem Bereich zwischen Zulauf und Seitenabzug das teilgespaltene Zwischenprodukt niedergeschlagen und in den Reaktionsbereich zur vollständigen Spaltung rückgeführt werden. Im Verstärkerteil oberhalb des Seitenabzuges findet die Trennung zwischen der Hydroxylkomponente und dem Isocyanat statt. Hier ist aufgrund der Rückreaktion ein möglichst niedriger Flüssigkeits-Hold-up anzustreben.

Zur Vermeidung von Rückreaktionen kann der Verstärker- und Mittelteil der Kolonne bei gegenüber dem Reaktionsteil in Abhängigkeit vom Sumpfdruck um bis zu 900 mbar vermindertem Druck betrieben werden, wobei die Trennung des Druckniveaus gegebenenfalls durch Abtrennung des Verstärker- und Mittelteils der Kolonne in einem gesonderten Apparat erfolgen kann.

Darüber hinaus kann der Rückreaktion auch durch einen nur im Verstärkerteil reduzierten Druck entgegengewirkt werden, der zu einer reduzierten Temperatur und damit zu verlangsamter Rückreaktion führt.

Als Apparatur sind Rektifizierkolonnen geeignet, die ein Abtriebsteil mit hinreichend hoher Verweilzeit und ein Verstärkerteil mit niedriger Verweilzeit aufweisen. Die Verweilzeit im Abtriebsteil ist auf die Spalt- und Stoffübergangskinetik abzustimmen und ist daher stark stoffsystemabhängig, sie beträgt 1 bis 1000 min, bevorzugt 5 bis 200 min und ist hier als Quotient von Flüssigkeits-Hold-up im Abtriebsteil und Zulauf-Volumenstrom der flüssigen Phase definiert. Druckverlustarme Packungen mit hohem Hold-up sind in Verbindung mit einem engsiedenden Lösemittel hierbei zu bevorzugen, da damit die Einstellung einer über den Reaktionsteil der Kolonne näherungsweise konstanten und über den Absolutdruck frei wählbaren Temperatur gelingt. Die Temperatur in der Reaktionszone ist bevorzugt in einem Temperaturbereich zu wählen, in dem einerseits die Reaktion ausreichend schnell verläuft und andererseits die Bildung von nicht recyclisierbaren Nebenprodukten noch nicht oder nur sehr schwach einsetzt.

Die Kolonne hat einen Rücklauf am Kolonnenkopf, mindestens einen Seitenabzug zur teilweisen oder vollständigen Entnahme der Flüssigphase sowie einen Sumpfablauf.

Als Verdampfer kommen alle gängigen Verdampfer zum Betreiben einer Kolonne in Betracht. Für einen dauerhaften Betrieb müssen die Heizflächen gut benetzt und umspült sein. Im Mittelteil und/oder im Abtriebsteil und/oder im Verstärkerteil der Kolonne sind als Einbauten Glockenböden, Siebböden oder geordnete bzw. ungeordnete Packungen möglich. Bevorzugt werden geordnete Packungen.

Wie bei den Verfahren der EP-A 54 817, EP-A 92 738 sowie EP-A 355 443 können die Spaltprodukte auch durch Dephlegmatoren getrennt werden, vorzugsweise werden sie durch Rektifikation getrennt.

Der Reaktorzulauf besteht aus dem Carbamidsäureester, gegebenenfalls einem Katalysator und/oder dem erfindungsgemäß verwendeten inerten Lösemittel sowie gegebenenfalls aus den Nebenprodukten, die in einem Kreislaufverfahren entstehen, bei dem aus einem Amin, einer Carbonylquelle wie beispielsweise Oxide des Kohlenstoffs oder Kohlensäurederivate, vorzugsweise Harnstoff und/oder Carbamidsäureester bzw. Dialkylcarbonate und einer Hydroxylkomponente ein Carbamidsäureester hergestellt wird. Das recyclisierte Lösemittel kann gegebenenfalls teilweise oder vollständig direkt in den Sumpf der Kolonne eingebracht werden.

Der Zulaufstrom der Spaltkolonne setzt sich allgemein zusammen aus dem Hauptstrom aus dem vorhergeschalteten Verfahrensschritt sowie gegebenenfalls a) dem nebenproduktarmen Teilstrom, der aus dem Sumpfaustrag der Spaltkolonne nach Ausschleusung von höhersiedenden Nebenprodukten resultiert und gegebenenfalls b) aus dem Sumpfaustrag der Reinkolonne sowie gegebenenfalls c) Frisch-Lösungsmittel und gegebenenfalls d) Kopfprodukt der Reinkolonne. Der Zulauf wird auf eine Temperatur vorgewärmt, welche bis zu 250°C oberhalb der Schmelztemperatur des Carbamidsäureesters, vorzugsweise 50°C unterhalb der Reaktionstemperatur liegt. Der Zulauf wird oberhalb des Abtriebsteils in die Kolonne eingespeist.

Zur Erhöhung der Reaktionsgeschwindigkeit kann die Spaltung der Carbamidsäureester in Gegenwart von Katalysatoren erfolgen. Normalerweise sind bei dem Verfahren Katalysatoren nicht erforderlich. Falls Katalysatoren zugesetzt werden, so werden zweckmäßigerweise Mengen bis 10 Gew.-%, vorzugsweise bis 3 Gew.-%, bezogen auf das Gewicht des Carbamidsäureesters verwendet. Als Katalysatoren eignen sich z.B. Metalle, Metalloxide, anorganische oder organische Metallverbindungen sowie saure Zusätze. Beispielhaft können die in der US-A 3 919 279, US-A 4 388 246, DE-A 3 277 748, DE-A 3 248 018, DE-A 3 314 790, US-A 4 873 365, EP-A 323 514, EP-A 126 299, EP-A 566 925 und EP-A 568 782 genannten Stoffe Verwendung finden.

Es kann auch durch geeignet wirkende Packungs- oder Füllkörperoberflächen heterogen katalysiert werden.

Die Spaltkolonne wird mit einem Sumpfdruck von 2 bis 1000 mbar betrieben, bevorzugt wird der Druckbereich von 20 bis 200 mbar. Die Sumpftemperatur beträgt 150 bis 400°C, bevorzugt 220 bis 300°C. Sie richtet sich im wesentlichen nach der Siedetemperatur des Lösemittels und ist zudem so zu wählen, daß Nebenreaktionen des Carbamidsäureesters nur schwach auftreten. Das Rücklaufverhältnis am Kopf liegt zwischen 0,2 und 20, bevorzugt 2 bis 10. Das Rücklaufverhältnis an der Seitenstromentnahme liegt zwischen 0 und 40, bevorzugt zwischen 5 und 20.

Der Sumpfaustrag dient zur Ausschleusung von Nebenprodukten sowie der Ausschleusung hochsiedender Verunreinigungen, die gegebenenfalls (z.B. zusammen mit dem Carbamidsäureester) zugefahren werden. Hierbei braucht nur die Menge an Lösemittel zu- bzw. abgeführt werden, wie zur Einhaltung einer vorgegebenen Nebenproduktkonzentration im Sumpf notwendig ist. Im Gegensatz zu den Verfahren der EP-A 0 524 554 und DE-A 42 31 417 erfolgt die vollständige Spaltung des Carbamidsäureesters normalerweise bereits beim ersten Spaltkolonnendurchlauf, so daß normalerweise kein Edukt im Kreis gefahren werden muß. In einem nachgeschalteten Apparat können hochsiedende Verunreinigungen aus dem Sumpfentnahmestrom auf an sich bekannte Weise, z.B. durch eine konventionelle Methode der Vakuumdestillation, vorzugsweise durch Dünnschichtdestillation und/oder durch Fallfilmdestillation ausgeschleust werden. Der lösemittelreiche Strom wird wieder auf die Spaltkolonne aufgegeben. Der isocyanatreiche Seitenstrom der Spaltkolonne wird anschließend einer Feindestillation unterworfen.

Der Sumpf der Feindestillation wird normalerweise dem Zulauf der Spaltkolonne beigemischt, bei einem größeren Anteil an höhermolekularen Reaktionsnebenprodukten kann der Sumpf auch ganz oder teilweise in die Stufe der Urethanherstellung zurückgeführt oder ausgeschleust werden.

### Beispiel

0,91 kg/h einer Mischung Hexamethylendi-n-butylurethan-1,6 (HDU-B) wurde zusammen mit 0,3 kg/h ortho-Phenoxybiphenyl (Gehalt nach GC >99 %, bei Betriebsdruck Siedebereich < 3°C) kontinuierlich in eine Spaltkolonne eingebracht. Die Aufgabe von HDU-B und von ortho-Phenoxybiphenyl erfolgte oberhalb des Abtriebsteils.

Die Kolonne besteht aus einem Verdampfer mit 4 waagerecht angeordneten Heizkerzen. Darüber befinden sich im Abtriebsteil auf einer Länge von 8,1 m eine geordnete Packung mit einem Durchmesser von 70 mm und einem Flüssigkeits-Hold-up von insgesamt rund 1500 ml. Oberhalb dieser Reaktionszone befindet sich der Zulauf, wobei das HDU-B mit 120°C und das Lösemittel mit 160°C zudosiert wird. Der Mittelteil der Kolonne hat 70 mm Durchmesser und ist über eine effektive Höhe von 990 mm mit Gewebe-Packungen bestückt. Darüber folgt die Seitenstrom-Entnahme und 850 mm effektive Gewebe-Packung mit 50 mm Durchmesser. Der Kopf der Kolonne besteht aus dem Flüssigkeitsteiler und einem wassergekühlten Kondensator. Die Kolonne ist isoliert. Das Kopfrücklaufverhältnis betrug im Beispiel 7, das Seitenstromrücklaufverhältnis 10. Der Kopfdruck betrug in diesem Beispiel 85 mbar, die Sumpftemperatur 260°C. Dem Kolonnensumpf wurden 0,3 kg/h Flüssigkeit entnommen, die gemäß Supercritcal Fluid Chromatography (SFC) nur den Wärmeträger enthielten, HDU-B oder dessen Folgeprodukte waren nicht nachweisbar (Nachweisgrenze 0,1 %). Den gleichen Befund erbrachte die IR-Analytik. Ebenso rein war die Flüssigkeit, die von der Destillationskolonne in den Sumpf abtropfte und von der Probe entnommen wurde, auch hier wurden weder über SFC- noch über IR-Analytik Verunreinigungen gefunden. Der Seitenstrom von 0,48 kg/h bestand aus 98,2 Gew.-% HDI, 1,6 Gew.-% halbgespaltenes Produkt der Formel (I)

BuOCONH-(CH₂)₆-NCO (I).

0,1 Gew.-% ortho-Phenoxybiphenyl und 0,1 Gew.-% BuOH. Der Kopf-Entnahmestrom betrug 0,43 kg/h und hatte eine Zusammensetzung von 99,5 % BuOH, 0,2 Gew.-% HDU-B und 0,3 Gew.-% (I). Die Ausbeute (maximal mögliche Menge HDI abzüglich Verlusten in Sumpf und Kopf) bei diesem Versuch betrug deutlich über 99 %. Hierbei ist der Gehalt an (I) im Seitenstrom nicht als Verlust gewertet, da (I) über den Rücklauf von der Reinkolonne zurückgewonnen werden kann. Die Ausbeute kann weiter erhöht werden, wenn das Kopfprodukt recyclisiert wird.

## Patentansprüche

1. Verwendung eines Lösemittels, welches einen definierten Siedepunkt (Reinstoff) oder aber einen engen Siedebereich < 10°C bei Betriebsdruck aufweist, und welches als Destillationsschnitt von thermostabilen Flüssigkeiten gewonnen wird, ausgewählt aus der Gruppe ortho-, meta- und para-Isomeren von Phenoxybiphenyl, zur thermischen Spaltung von Carbamidsäureester.

2. Verwendung gemäß Anspruch 1, **dadurch gekennzeichnet, daß** als Hochsieder Destillationsschnitte handelsüblicher Phenoxybiphenyl und o-Phenoxybiphenyl Wärmeträger mit einem Siedebereich von < 3°C (bei Betriebsdruck) eingesetzt werden.

3. Verwendung nach Anspruch 1, **dadurch gekennzeichnet, daß** die Spaltung in einer Reaktivreaktifikationskolonne durchgeführt wird.

## Claims

1. The use of a solvent which has a defined boiling point (pure substance) or which has a narrow boiling range <10°C at the operating pressure, and which is obtained as a distillation cut from thermally stable liquids selected from the group ortho-, meta- and para-isomers of phenoxybiphenyl, for the thermal cracking of carbamidic acid esters.

2. A use according to claim 1, **characterised in that** distillation cuts of commercially available phenoxybiphenyl and o-phenoxybiphenyl heat transfer media with a boiling range < 3°C (at the operating pressure) are used as high-boiling solvents.

3. A use according to claim 1, **characterised in that** cracking is conducted in a reactive rectification column.

## Revendications

1. Utilisation d'un solvant à point d'ébullition défini (substance pure) ou à intervalle d'ébullition étroit, inférieur à 10°C à la pression opératoire, obtenu en coupe de distillation de liquides stables à la chaleur, et choisi dans le groupe des isomères ortho, méta et para du phénoxybiphényle, pour la scission des esters carbamiques à la chaleur.

2. Utilisation selon la revendication 1, **caractérisée en ce que** l'on utilise en tant que produits à haut point d'ébullition des coupes de distillation du phénoxybiphényle et de l'o-phénoxybiphényle du commerce, agents d'échange de chaleur à un intervalle d'ébullition inférieur à 3°C (à la pression opératoire).

3. Utilisation selon la revendication 1, **caractérisée en ce que** la scission est réalisée dans une colonne de rectification réactive.
